(19)

Europäisches
Patentamt
European
Patent Office
Office européen
des brevets

(11)  **EP 4 437 956 A1**

(12)  **EUROPEAN PATENT APPLICATION**
published in accordance with Art. 153(4) EPC

(43) Date of publication:
**02.10.2024 Bulletin 2024/40**

(21) Application number: **23740418.1**

(22) Date of filing: **09.01.2023**

(51) International Patent Classification (IPC):
**A61B 5/1495** (2006.01)   **A61B 5/145** (2006.01)
**A61B 5/155** (2006.01)

(52) Cooperative Patent Classification (CPC):
**A61B 5/145; A61B 5/1495; A61B 5/155**

(86) International application number:
**PCT/KR2023/000397**

(87) International publication number:
**WO 2023/136574 (20.07.2023 Gazette 2023/29)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **17.01.2022 KR 20220006625**

(71) Applicant: **i-Sens, Inc.**
**Seoul 06646 (KR)**

(72) Inventors:
• **NAH, Ji Seon**
  **Seoul 06646 (KR)**
• **KANG, Young Jea**
  **Seoul 06646 (KR)**
• **SEO, Jung Hee**
  **Seoul 06646 (KR)**
• **JANG, Min Ji**
  **Seoul 06646 (KR)**
• **KANG, Yun Hee**
  **Seoul 06646 (KR)**

(74) Representative: **BCKIP Part mbB**
**Siegfriedstraße 8**
**80803 München (DE)**

(54)  **METHOD FOR CORRECTING BIOMETRIC SIGNALS IN CONTINUOUS BIOMETRIC INFORMATION MEASUREMENT SYSTEM**

(57)   The present invention relates to a method for correcting biometric signals in a continuous biometric information measurement system and, more specifically, to a method which, if a correction parameter is unstable when a correction factor is being calculated using a reference biometric value, does not use the current correction factor calculated from a reference biometric value as-is, but revises the current correction factor by applying, to the current correction factor, a compensation value calculated from the difference between the current correction factor and a previous correction factor, and thus even if a correction parameter is unstable it is possible to calculate a corrected biometric value by accurately correcting a biometric signal. The method also divides the entire usage time period of a sensor into multiple correction periods, and revises the correction factor in consideration of the degree of stabilization of the sensor depending on the correction period to which the time point at which the reference biometric value is input belongs, so as to accurately calculate the corrected biometric value from the biometric signal from the sensor.

Fig. 4

## Description

## TECHNICAL FIELD

The present disclosure relates to a method for calibrating biometric signals in a continuous biometric information measurement system and, more specifically to a method in which, when calculating a calibration factor using a reference biometric value, if a calibration parameter is unstable, the current calibration factor calculated from the reference biometric value is not used as is, but as the current calibration factor is corrected by applying a compensation value, which is calculated from the difference between the current calibration factor and the previous calibration factor, to the current calibration factor, even if the calibration parameter is unstable, it is possible to calculate a calibrated biometric value by accurately calibrating biometric signals, and as the entire usage period of the sensor is divided into a number of calibration sections and the calibration factor is corrected by considering the degree of stabilization of the sensor according to the calibration section to which the reference biometric value is input, it is possible to accurately calculate the calibrated biometric value from the biometric signals of the sensor.

## BACKGROUND

Diabetes is a chronic medical condition that is common in modern people, and in the Republic of Korea, there are 2 million diabetes patients, about 5% of the total population.

Diabetes occurs when the absolute level of the sugar level in blood is high due to the absolute deficiency or relative insufficiency of insulin, produced by the pancreas, caused by various reasons such as obesity, stress, poor eating habits, and inherited hereditary factors and imbalance regarding glucose in the blood.

The blood usually contains a certain concentration of glucose, and tissue cells gain energy from the glucose.

However, when the glucose is increased excessively more than needed, the glucose cannot be properly stored in the liver, muscle, or adipose tissue and is accumulated in the blood, because of this, patients with diabetes maintain a much higher blood glucose level than normal people, and as excessive blood glucose passes through the tissues and is discharged into the urine, it results in deficiency of glucose, which is absolutely necessary for all tissues of the body, thereby causing abnormalities in respective body tissues.

Diabetes is characterized by substantial absence of subjective symptoms at the beginning of the condition, when diabetes progresses, diabetes-specific symptoms such as overdrink, overeat, polyuria, weight loss, weariness, skin itchiness, and lower ability of naturally healing on injury on hands and feet are shown, and further progression of diabetes leads to complications such as visual disturbances, hypertension, kidney disease, paralysis, periodontal disease, muscle spasms and neuralgia, as well as gangrene.

In order to diagnose diabetes beforehand and manage to prevent the progression of diabetes into complications associated therewith, systematic blood glucose measurement and treatment should be performed.

Diabetes needs to constantly measure blood glucose for management, so the demand for devices related to blood glucose measurement is steadily increasing. It has been confirmed through various studies that, when diabetic patients strictly control the management of blood glucose, the incidence of complications of diabetes is significantly reduced. Accordingly, it is very important for diabetic patients to measure blood glucose regularly for blood glucose management.

The finger prick method is generally used to manage blood glucose level in diabetic patients, and although such a blood collecting glucose measurement device helps diabetics patients manage blood glucose level, it is difficult to accurately identify frequently changing blood glucose levels because only the results at the time of measurement appear. In addition, the finger prick method requires blood collection to measure blood glucose levels frequently throughout the day, which poses a significant burden on diabetic patients.

Diabetics patients generally experience hyperglycemia and hypoglycemia, and an emergency may occur in the hypoglycemic conditions. Hypoglycemia occurs when sugar content is not kept for a long time, and the patients may become unconscious or die in the worst case. Accordingly, rapid discovery of the hypoglycemic condition is critically important for diabetics. The blood-gathering-type biometric information measuring device intermittently measuring glucose has limited ability to accurately measure blood glucose levels.

Recently, to overcome such a drawback of the blood-collecting-type biometric information measuring device, continuous glucose monitoring systems (CGMSs) inserted into the human body to measure a blood glucose level every few minutes have been developed, and therefore easily perform the management of diabetics and responses to an emergency situation.

The continuous blood glucose monitoring system includes a sensor transmitter that measures biometric values from body fluids by being attached to the body of a user, and a communication terminal that outputs information about the received biometric values to a user. The sensor transmitter includes a sensor for continuous blood glucose measurement that is partially inserted into the human body, and the sensor is inserted into the human body for a certain period of use, for example, approximately 15 days. The sensor transmitter periodically measures biometric values from body fluids, and a biometric management application is installed in the communication terminal to periodically receive biometric values from the sensor transmitter and output the information about the received biometric values to a user.

The sensor of the sensor transmitter is continuously inserted into a skin throughout an usage period, and the sensitivity of the sensor may vary depending on the part of the body where the sensor is inserted, and even if the sensor insertion location on the body part is the same, the sensitivity of the sensor may change over time. Biometric values measured by a sensor transmitter have errors according to changes in sensitivity, and the biometric values of a user must be calibrated by applying calibration factor to the measured biometric values to overcome the errors.

In order to provide accurate biometric values to a user, the biometric values received from the sensor transmitter must be initially calibrated and then continuously calibrated at a certain calibration cycle during the usage period of the sensor transmitter. More specifically, at each calibration cycle during the period of use of the sensor transmitter, the calibration factor is calculated using the reference biometric values measured by a separate measuring device and the biometric values received from the sensor transmitter, and the biometric values received from the sensor transmitter must be calibrated by applying a calibration factor before the next calibration cycle arrives.

However, when the reference biometric values are measured while the calibration parameters such as change rate and size of the biometric values measured by the sensor are unstable, and the calibration factor is calculated using the reference biometric values measured at this time, there is a problem in that calibrated biometric values cannot be accurately determined.

Meanwhile, it takes a certain amount of time for the sensor to stabilize after being inserted into the body, and before the sensor stabilizes, the reliability of the measured biometric values is relatively low, and as the sensor becomes more stable, the reliability of the measured biometric values relatively increases. Therefore, when calculating the calibration factor using reference biometric values measured in a state in which the calibration parameter is unstable, it is necessary to calculate the calibration factor differently by considering the degree of stabilization of the sensor.

**SUMMARY**

**Technical Problem**

As the present disclosure is intended to solve the problems of the conventional calibration factor calculation method mentioned above, the purpose of the present disclosure is to provide a method of accurately calculating a calibration factor depending on whether a calibration parameter is unstable when calculating the calibration factor using reference biometric values.

Another purpose of the present disclosure is to provide a method of accurately calculating the calibration factor by dividing the entire usage period of the sensor into multiple calibration sections and considering the degree of stabilization of the sensor according to the calibration section to determine the calibration factor, if the calibration parameters are unstable when calculating the calibration factor using reference biometric values.

**Solution to Problem**

To accomplish the purpose of the present disclosure described above, a method for calibrating a biometric signal according to the present disclosure may comprise: determining whether a reference biometric value used to calculate a calibrated biometric value from a biometric signal measured by a sensor is input; extracting a calibration parameter at a time point when the reference biometric value is input, and determining whether a correction condition is satisfied based on the extracted calibration parameter; and calculating a calibration factor depending on whether the correction condition is satisfied and calculating the calibrated biometric value from the biometric signal measured by the sensor using the calibration factor.

According to the method for calibrating the biometric signal according to the present disclosure, if the correction condition is not satisfied, a current calibration factor may be calculated from the input reference biometric value, and the calibrated biometric value is calculated from the biometric signal using the current calibration factor until a next reference biometric value is input.

Preferably, the method for calibrating the biometric signal according to the present disclosure may comprise: if the correction condition is satisfied, calculating a current correction factor from the input reference biometric value; determining a calibration section to which the time point when the reference biometric value is input belongs; calculating a final calibration factor from the current calibration factor differently depending on the calibration section to which the time point when the reference biometric value is input belongs, based on the determined calibration section; and calculating a calibrated biometric value using the biometric signal measured by the sensor until the next reference biometric value is input and the final calibration factor.

In the method for calibrating the biometric signal according to the present disclosure, the calibration section may divide an entire usage period of the sensor into a first section, a second section, and a third section over time based on time of inserting the sensor into a body.

Here, the current calibration factor is calculated as the final calibration factor when the time point when the reference biometric value is input is within the first section.

Here, when the calibration section to which the time point when the reference biometric value is input is within the second section, a first compensation value is calculated from a difference between the current calibration factor and a previous calibration factor, and the final calibration factor is calculated by applying the first compensation value to the current calibration factor.

Here, when the calibration section to which the time point when the reference biometric value is input is within the third section, a second compensation value is calculated from a difference between the current calibration factor and the previous calibration factor, and the final calibration factor is calculated by applying the second compensation value to the current calibration factor.

Preferably, the method for calibrating the biometric signal according to the present disclosure may comprise: calculating an average value of previous final calibration factors calculated in the third section based on the time point when the reference biometric value is input; and re-correcting the final calibration factor based on the average value.

Preferably, the re-correcting of the final calibration factor according to an embodiment comprises: calculating a correction threshold range based on the average value; determining whether the final calibration factor is out of the correction threshold range; and re-correcting the final calibration factor, depending on whether the final calibration factor is out of the correction threshold range, and the calibrated biometric value is calculated using the re-corrected final calibration factor.

Here, if the final calibration factor is out of the correction threshold range, the final calibration factor is re-corrected to the correction threshold range.

Here, if the final calibration factor is within the correction threshold range, the final correction factor is used as is.

Preferably, the re-correcting of the final calibration factor according to another embodiment comprises calculating a difference between the final calibration factor and the average value; calculating a third compensation value from the difference between the final calibration factor and the average value; and re-correcting the final calibration factor with the third compensation value, wherein the calibrated biometric value is calculated using the re-corrected final calibration factor.

Here, the sensor is configured to be inserted into the body of a user and continuously measure the biometric signal from body fluid for a certain period of time.

Here, the sensor is configured to measure a blood glucose value of a user, and the reference biometric value is a reference blood glucose value measured by a separate blood glucose meter for calibrating the measured blood glucose value measured by the sensor.

## Advantageous Effects of Invention

In the method of calibrating a biometric signal according to the present disclosure, when calculating a calibration factor using a reference biometric value, if the calibration parameter is unstable, the current calibration factor calculated from the reference biometric value is not used as is, but as the current calibration factor is corrected by applying a compensation value, which is calculated from the difference between the current calibration factor and the previous calibration factor, to the current calibration factor, even if the calibration parameter is unstable, it is possible to calculate a calibrated biometric value by accurately calibrating biometric signals

Additionally, in the method for calibrating biometric signals according to the present disclosure, when calculating calibration factors using reference biometric values, if the calibration parameters are unstable, the entire usage period of the sensor is divided into a number of calibration sections and the calibration factor is corrected by considering the degree of stabilization of the sensor according to the calibration section to which the reference biometric value is input, it is possible to accurately calculate the calibrated biometric value from the biometric signals of the sensor.

Additionally, the biometric signal calibration method according to the present disclosure calculates the average value of the calibration factor in the section where the sensor is stabilized, and by re-correcting the calibration factor, considering the difference between the corrected calibration factor and the average value, it is possible to accurately calculate the calibrated biometric value from the biometric signal of the sensor in the section where the sensor is stabilized.

## BRIEF DESCRIPTION OF THE DRAWINGS

FIG. 1 is a schematic diagram showing a continuous biometric value measurement system according to an embodiment of the present disclosure.

FIG. 2 is a functional block diagram for explaining the device for calibrating blood glucose signals according to the present disclosure.

FIG. 3 is a functional block diagram for explaining the calibration unit according to the present disclosure.

FIG. 4 is a flowchart illustrating the method for calibrating a blood glucose signal according to the present disclosure.

FIG. 5 is a diagram for explaining an example of inputting calibration information according to a calibration cycle.

FIG. 6 shows an example of a calibration section that divides the entire usage period of the sensor according to the present disclosure.

FIG. 7 is a flowchart illustrating an example of a method for calculating a calibration factor in the present disclosure.

FIG. 8 is a flowchart to explain an example of the step of re-correcting the corrected calibration factor in the present disclosure.

FIG. 9 is a flowchart to explain another example of the step of re-correcting the corrected calibration factor in the present disclosure.

**DETAILED DESCRIPTION OF EMBODIMENTS**

It should be noted that the technical terms used in the present disclosure are only used to describe specific embodiments and are not intended to limit the present disclosure. In addition, the technical terms used in the present disclosure, unless specifically defined in a different sense in the present disclosure, should be interpreted as meanings generally understood by those skilled in the art in the technical field to which the present disclosure belongs, and should not be interpreted as an excessively comprehensive sense or an excessively reduced sense. In addition, if the technical term used in the present disclosure is an incorrect technical term that does not accurately express the idea of the present disclosure, it should be replaced with a technical term that can be correctly understood by a person skilled in the art.

Additionally, as used in the present disclosure, singular expressions include plural expressions unless the context clearly dictates otherwise. In the present disclosure, terms such as "consists of" or "comprises" should not be construed as necessarily including all of the various components or steps described in the disclosure and should be interpreted that some of the components or steps may not be included, or additional components or steps may be included.

In addition, it should be noted that the attached drawings are only intended to facilitate easy understanding of the concepts of the present disclosure, and should not be construed as limiting the concepts of the present disclosure by the attached drawings.

Hereinafter, the calibration alarm method according to the present disclosure will be examined in more detail with reference to the attached drawings.

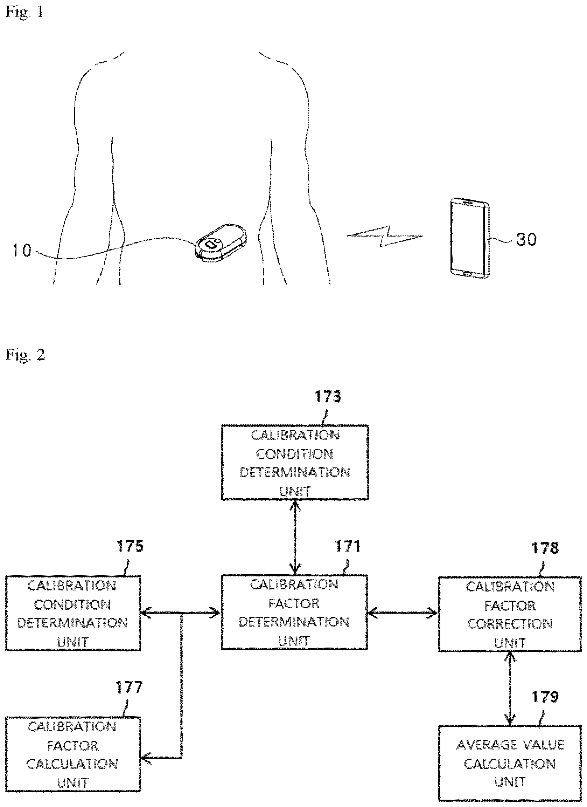

FIG. 1 is a schematic diagram showing a continuous biometric value measurement system according to an embodiment of the present disclosure.

Hereinafter, a blood glucose value will be described as an example of a biometric value, and a reference blood glucose value will be described as an example of a reference biometric value, but various biometric values other than a blood glucose value can be measured depending on the field to which the present disclosure is applied.

Referring to FIG. 1, a continuous biometric value measurement system (1) according to an embodiment of the present disclosure includes a sensor transmitter (10) and a communication terminal (30).

The sensor transmitter (10) is attached to a body, and when the sensor transmitter (10) is attached to the body, one end of the sensor of the sensor transmitter (10) is inserted into a skin and periodically measures biometric signals representing blood glucose levels, that is, blood glucose signals, from body fluids of the human body.

The communication terminal (30) is a terminal that may receive a blood glucose signal from the sensor transmitter (10), calibrate the received blood glucose signal with a calibration factor, convert the unit into a calibrated blood glucose value, and then display it to a user, so a terminal capable of communicating with the sensor transmitter (10), such as a smartphone, tablet PC, or laptop, may be used. Of course, the communication terminal (30) is not limited thereto, and may be any type of terminal may be used as long as it includes a communication function and a program or application may be installed.

That is, the sensor transmitter (10) generates a blood glucose signal, for example, a current signal, corresponding to the blood glucose value of a user and transmits the blood glucose signal to the communication terminal (30), and the communication terminal (30) calibrates the blood glucose signal of the current value with a calibration factor and converts the unit into a calibrated blood glucose value. Depending on the field to which the present disclosure is applied, the sensor transmitter (10) can directly convert units from a blood glucose signal to a blood glucose value, and the communication terminal (30) can calibrate the blood glucose value received from the sensor transmitter (10) with a calibration factor.

Hereinafter, in the present disclosure, the blood glucose signal or the unit-converted blood glucose value measured by the sensor transmitter (10) is referred to as a measured blood glucose value, and the blood glucose value obtained by calibrating the blood glucose signal or the unit-converted blood glucose value with a calibration factor in the communication terminal (30) is referred to as the calibrated blood glucose value.

The sensor transmitter (10) transmits information about the measured blood glucose value to the communication terminal (30) at the request of the communication terminal (30) or at a set time, and for data communication between the sensor transmitter (10) and the communication terminal (30), the sensor transmitter (10) and the communication terminal (30) may be connected to each other through a wired communication method such as a USB cable or a wireless communication method such as infrared communication, NFC communication, or Bluetooth.

When communication is connected between the sensor transmitter (10) and the communication terminal (30), the initial calibration factor is calculated using the reference blood glucose value measured by a separate blood glucose meter (not shown) after the initial stabilization of the sensor transmitter (10), and an initial calibration is performed on the measured blood glucose value using the initial calibration factor. Thereafter, the communication terminal (30) calibrates the measured blood glucose value received from the sensor transmitter (10) with an initial calibration factor, and provides the calibrated blood glucose value to a user.

Here, the initial stabilization may be determined to be initially stabilized after a certain period of time, for example, 2 hours after the sensor is inserted into a body, or may be determined based on the measured blood glucose value. However, the initial stabilization requires additional time, for example 3 to 10 days, for the sensor to actually be stabilized as the minimum stabilization time or conditions required to provide a user with a calibrated blood glucose value as soon as possible after inserting the sensor into a body.

In order to accurately calibrate the blood glucose value measured in the sensor transmitter (10) after the initial stabilization, the communication terminal (30) calculates a new calibration factor using the reference blood glucose value measured by a separate blood glucose meter at each calibration cycle during the usage period of the sensor transmitter (10). Then by calibrating the measured blood glucose value received from the sensor transmitter using a new calibration factor, the calibrated blood glucose value is calculated, and the calculated calibrated blood glucose value is output to the user.

Preferably, the calibration cycle can be changed depending on the progress of stabilization. For example, after initial stabilization, calibration can be performed at 12-hour intervals, and thereafter, calibration can be performed at intervals of 24 hours or 48 hours depending on the progress of stabilization.

FIG. 2 is a functional block diagram for explaining the device for calibrating blood glucose signals according to the present disclosure.

The device for calibrating blood glucose signals may be implemented in a communication terminal such as a smart terminal that communicates with the sensor transmitter and notifies the calibrated blood glucose value to a user, or may be implemented through a separate receiving device.

Looking more specifically with reference to FIG. 2, a calibration alarm unit (130) provides a calibration alarm to a user through a user interface unit (110) when a calibration cycle arrives according to the calibration cycle stored in a storage unit (150).

Preferably, the calibration alarm unit (130) extracts the calibration parameters stored in the storage unit (130) when the calibration cycle arrives, and by comparing the extracted calibration parameter with the calibration limit condition, if the calibration limit condition is satisfied, a calibration alarm is not provided to the user interface unit (110) or a calibration icon that allows input of calibration commands can be deactivated on the user interface unit (110). Here, the calibration parameters are the calibrated blood glucose value, the rate of change of the calibrated blood glucose value, and the speed of change of the calibrated blood glucose value, and the calibration limit condition may be whether the calibrated blood glucose value exceeds the threshold limit of the blood glucose value or whether the rate of change of the calibrated blood glucose value exceeds the threshold limit of the rate of change.

In a case in which a reference blood glucose value is entered through the user interface unit (110), a calibration unit (170) determines the calibration section to which the input time of the reference blood glucose value belongs or determines whether the calibration parameter at the time the reference blood glucose value is input satisfies the correction condition. The calibration unit (170) calculates a calibration factor based on whether the calibration parameter satisfies the correction condition and the calibration section to which the input time of the reference blood glucose value belongs, and calculates the calibrated blood glucose value using the calculated calibration factor.

When the reference blood glucose value is input, the calibration unit (170) determines the measured blood glucose value corresponding to the input time of the reference blood glucose value among the measured blood glucose values received through the transceiver unit (190) and creates a calibration pair consisting of the reference blood glucose value and the corresponding measured blood glucose value. The calibration unit (170) calculates the calibration factor using the calibration pair. Depending on the field to which the present disclosure is applied, the calibration unit (170) may calculate the calibration factor using the current calibration pair consisting of the entered reference blood glucose value and the corresponding measured blood glucose value, but in order to calculate the accurate calibration factor, a calibration factor may be calculated with a regressive method using the current calibration pair and the past calibration pairs.

The calibration unit (170) may use the calculated calibration factor as is or correct the calculated calibration factor based on whether the calibration parameter satisfies the correction condition at the time the reference blood glucose value is entered or the calibration section to which the input time of the reference blood glucose value belongs. The calibration unit (170) calculates a calibrated blood glucose value by calibrating the measured blood glucose value, which is received after the reference blood glucose value is input, with a calibration factor, and provides the calculated calibrated blood glucose value to a user through the user interface unit (110).

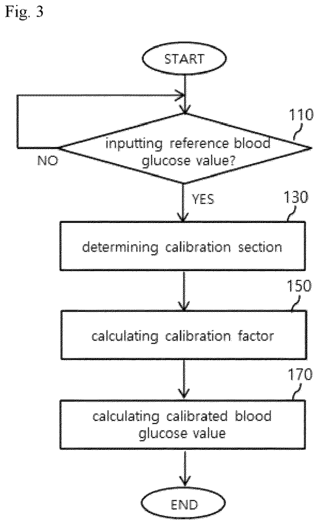

FIG. 3 is a functional block diagram for explaining the calibration unit according to the present disclosure.

Looking in more detail with reference to FIG. 3, when the reference blood glucose value is input, a calibration section determination unit (173) determines the calibration section to which the input time of the reference blood glucose value belongs based on the time when the reference blood glucose value is input.

Meanwhile, a calibration condition determination unit (175) extracts the calibration parameter at the time the reference blood glucose value is input and determines whether the calibration parameter satisfies the correction condition, and a calibration factor calculation unit (177) calculates the current calibration factor from the current calibration pair or the past calibration pair.

A calibration factor determination unit (171) determines the current calibration factor calculated by the calibration factor calculation unit (177) as the calibration factor or determines the corrected calibration factor as the calibration factor by allowing a calibration factor correction unit (178) to correct the current calibration factor based on whether the calibration parameter satisfies the correction condition at the calibration section to which the reference blood glucose value is input and at the time the reference blood glucose value is input.

In a case in which the correction parameter satisfies the correction condition at the time the reference blood glucose value is input, the correction factor determination unit (171) requests the calibration factor correction unit (178) to correct the current correction factor. When receiving a request for correction of the current correction factor, the calibration factor correction unit (178) calculates a compensation value based on the calibration section to which the input time of the reference blood glucose value belongs and the difference between the current calibration factor and the previous calibration factor, and then the calculated compensation value is applied to the current calibration factor to calculate the corrected calibration factor.

According to the progress of stabilization, when the degree of stabilization of the sensor is high, it is necessary to maintain the calibration factor to not change significantly, so when it is determined that the time at which the reference blood glucose value is input is in the calibration section having high stabilization of the sensor, the calibration factor correction unit (178) may re-correct the corrected calibration factor based on the average value of the previous calibration factor calculated by an average value calculation unit (179) in the calibration section where the sensor has high stabilization.

In this way, based on whether the calibration parameter at the time the reference blood glucose value is input satisfies the correction condition, if the correction condition is not satisfied, the calibration factor determination unit (171) uses the current calibration factor calculated. However, if the calibration parameter at the time the reference blood glucose value is input satisfies the correction condition, the current calibration factor is corrected by applying a differently calculated compensation value depending on the calibration section to which the input time of the reference blood glucose value belongs, or the corrected calibration factor can be re-corrected based on the average value of the previous calibration factor in the calibration section where the sensor is highly stable.

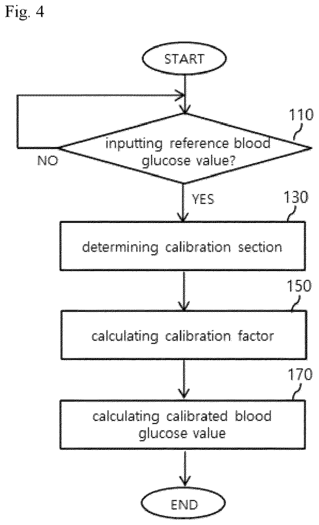

FIG. 4 is a flowchart illustrating the method for calibrating a blood glucose signal according to the present disclosure.

Looking more specifically with reference to FIG. 4, it is determined whether a calibration cycle has arrived or whether a reference blood glucose value is entered by user request regardless of the calibration cycle (S 110).

When the reference blood glucose value is input, the calibration section to which the input time of the reference blood glucose value belongs in the entire usage period of the sensor is determined (S 130). Here, the calibration section may divide the entire usage period of the sensor into a first section, a second section, and a third section over time based on the time of insertion of the sensor into the body. The first section refers to a section in which the degree of stabilization of the sensor is low after sensor insertion, the second section refers to a section in which the degree of stabilization of the sensor has further progressed after the first section, and the third section refers to a section in which the degree of stabilization of the sensor is higher than the second section after the second section. Depending on the field to which the present disclosure is applied, the entire usage period of the sensor can be divided into a number of calibration sections, which fall within the scope of the present disclosure.

The calibration factor is calculated based on the calibration section to which the input time of the reference blood glucose value belongs (S150). Then, the measured blood glucose value is calibrated using the calculated calibration factor to calculate the calibrated blood glucose value, and the calculated calibrated blood glucose value is provided to a user (S170).

The calibration factor may be calculated differently depending on whether the calibration parameter extracted at the time the reference blood glucose value is input satisfies the correction condition in the calibration section to which the input time of the reference blood glucose value belongs.

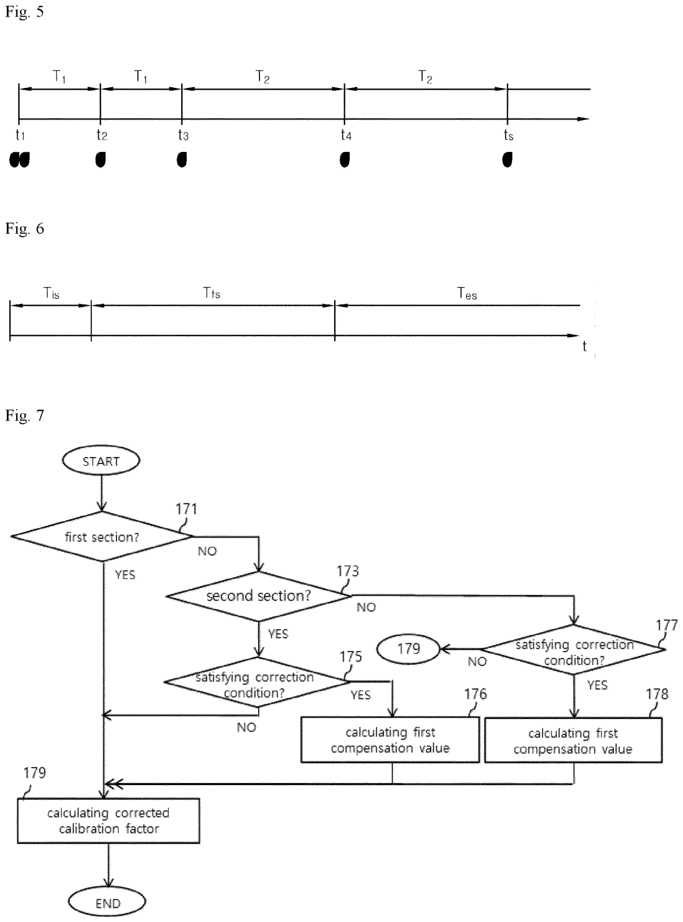

FIG. 5 is a diagram illustrating an example of inputting calibration information according to a calibration cycle, and here, the calibration information is the reference blood glucose value of a user measured by a test strip through a separate blood glucose meter.

Referring to FIG. 5, at the time when the sensor of the sensor transmitter is initially stabilized (t1), initial calibration information is input to the communication terminal. Here, the initial calibration information may be entered multiple times to accurately calculate the calibration factor. The communication terminal calculates the initial calibration factor using the initial calibration information and the blood glucose value measured by the sensor transmitter, and calculates the calibrated blood glucose value by calibrating the measured blood glucose value received from the sensor transmitter using the initial calibration factor.

After the sensor of the sensor transmitter is initially stabilized, new calibration information is periodically input to the communication terminal, preferably at a calibration cycle of 12 hours, 24 hours, etc., until the expiration of the usage period of the sensor transmitter, and after the sensor of a sensor transmitter has initially stabilized, new calibration information can be input to the sensor at a calibration cycle (T1) of every 12 hours for a certain period of time, and then new calibration information can be input to the sensor of the sensor transmitter at a second calibration cycle (T2) of 24 hours, 48, etc.

When a sensor is manufactured in the same environment and under the same conditions, the sensor has a certain sensitivity change characteristic (sensitivity drift) after insertion into a human body, and based on the sensitivity change characteristic (sensitivity drift), the calibration cycle can be set differently depending on the sensor manufacturing environment.

FIG. 6 shows an example of a calibration section that divides the entire usage period of the sensor according to the present disclosure.

Referring to FIG. 6, the first section ($T_{is}$) is a section in which the degree of stabilization of the sensor is low after initial stabilization, The second section ($T_{fs}$) is a section in which the stabilization information of the sensor is in the middle after the first section has elapsed, and the third section ($T_{es}$) is a section in which the degree of stabilization of the sensor is higher than the second section after the second section has elapsed.

Depending on the field to which the present disclosure is applied, the first section can be set to 0.5 to 1 day from the time the sensor is inserted into the body, the second section can be set to 3 to 5 days from the end of the first section, and the third section can be set from the end of the second section to the remaining usage period of the sensor. Depending on the field to which the present disclosure is applied, the first section, second section, and third section may be set to different times, which fall within the scope of the present disclosure.

FIG. 7 is a flowchart illustrating an example of a method for calculating the calibration factor in the present disclosure.

Looking more specifically with reference to FIG. 7, it is determined whether the calibration section to which the input time of the reference blood glucose value belongs is the first section (S171). If the calibration section to which the input time of the reference blood glucose value belongs is the first section, the current calibration factor calculated from the reference blood glucose value and the measured blood glucose value corresponding to input time of the reference blood glucose value is calculated as the corrected calibration factor (S179).

Meanwhile, if the calibration section to which the input time of the reference blood glucose value belongs is not the first section, it is determined whether the calibration section to which the input time of the reference blood glucose value belongs is the second section (S173). If the calibration section to which the input time of the reference blood glucose value belongs is the second section, it is determined whether the calibration parameter extracted at the time the reference blood glucose value is input satisfies the correction condition (S175). If the calibration section to which the input time of the reference blood glucose value belongs is the second section, and at this time, the calibration parameters do not satisfy the correction conditions, the current calibration factor is calculated as the corrected calibration factor (S179). However, if the calibration section to which the input time of the reference blood glucose value belongs is the second section and at this time, the calibration parameter satisfies the correction condition, the first compensation value is calculated from the difference between the current calibration factor and the previous calibration factor, and the first compensation value is applied to the current calibration factor to calculate the corrected calibration factor (S179).

For example, if the calibration section to which the input time of the reference blood glucose value belongs is the second section and at this time, the calibration parameter satisfies the correction condition, the corrected calibration factor can be calculated as in Equation (1) below.

[Equation 1]

$$CF_R = CF_C - (CF_C - CF_P) \times \alpha_1$$

Here, $CF_R$, $CF_C$, $CF_P$, $\alpha_1$ refer to the corrected calibration factor, current calibration factor, previous calibration factor, and first weight, respectively, and $(CF_C - CF_P) \times \alpha_1$ refers to the first compensation value.

Here, the first weight can be set between 0.3 and 0.6.

Meanwhile, if the calibration section to which the input time of the reference blood glucose value belongs is not the second section, the calibration section to which the input time of the reference blood glucose value belongs is determined to be the third section, and it is determined whether the extracted calibration parameter which is extracted at the time the reference blood glucose value is entered satisfy the correction conditions (S177). If the calibration section to which the input time of the reference blood glucose value belongs is the third section, and at this time the calibration parameter does not satisfy the correction conditions, the current calibration factor is calculated as the corrected calibration factor (S179). However, if the calibration section to which the input time of the reference blood glucose value belongs is the third section and the calibration parameter at this time satisfies the correction conditions, the second compensation value is calculated from the difference between the current calibration factor and the previous calibration factor, and a corrected calibration factor is calculated by applying the second compensation value to the current calibration factor (S179).

For example, if the calibration section to which the input time of the reference blood glucose value belongs is the third section, and at this time the calibration parameter satisfies the correction conditions, the corrected calibration factor can be calculated as shown in Equation (2) below.

[Equation 2]

$$CF_R = CF_C - (CF_C - CF_P) \times \alpha_2$$

Here, CFR, CFC, CFP, and $\alpha_2$ refer to the corrected calibration factor, current calibration factor, previous calibration factor, and the second weight, respectively, and $(CF_C - CF_P) \times \alpha_2$ refers to the second compensation value.

Here, the second weight may be set to 0.6 to 0.9.

Here, the correction condition is a condition for determining a calibration parameter in a range outside the normal range, and may be set differently or identically depending on the calibration section.

For example, an example of the correction condition is as follows.

1) When the reference blood glucose value exceeds the upper threshold blood glucose value and at the same time the current calibration factor is greater than the previous calibration factor

2) When the reference blood glucose value is less than the lower threshold blood glucose value and the previous calibration factor is larger than the current calibration factor

3) When the rate of change of increase in blood glucose value at the time the reference blood sugar value is input is greater than the threshold rate of change and the current calibration factor is greater than the previous calibration factor.

4) When the rate of change of decrease in blood glucose value at the time the reference blood glucose value is input is greater than the threshold rate of change and the previous calibration factor is greater than the current calibration factor.

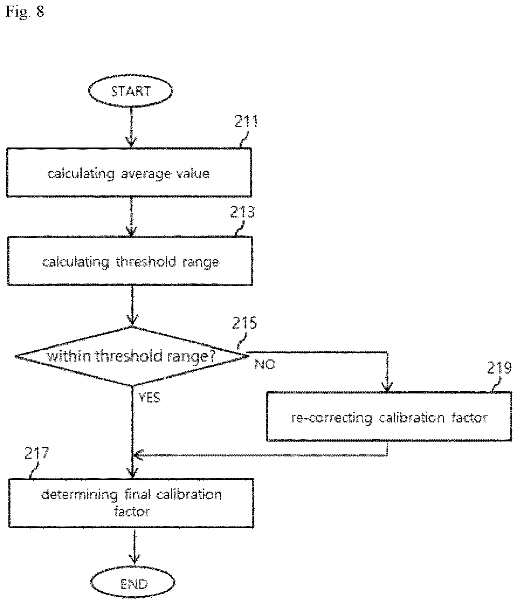

FIG. 8 is a flowchart to explain an example of the step of re-correcting the corrected calibration factor in the present disclosure.

If the calibration section to which the input time of the reference blood glucose value belongs is the third section, the calibration factor needs to be maintained so that it does not change significantly because the degree of stabilization of the sensor is high in the third section. So the corrected calibration factor can be re-corrected to follow the average value of the calibration factor calculated in the third section.

Looking more specifically with reference to FIG. 8, the average value of the corrected calibration factor calculated in the third section is calculated based on the time when the reference biometric value is input (S211), and the correction threshold range is calculated based on the calculated average value (S213).

It is determined whether the corrected calibration factor is outside the correction threshold range (S215), and the final calibration factor is determined by re-correcting the corrected calibration factor depending to whether the corrected calibration factor is outside the correction threshold range (S217).

That is, if the corrected calibration factor is in the correction threshold range, the corrected calibration factor is determined as the final calibration factor. However, if the corrected calibration factor is outside the correction threshold range, the final calibration factor is determined by re-correcting the corrected calibration factor into the correction threshold range (S219). Once the final calibration factor is determined, the calibrated biometric value is calculated using the final calibration factor.

For example, if the corrected calibration factor exceeds the upper limit of the correction threshold range, the corrected calibration factor is re-corrected to the upper limit of the correction threshold range, and if the corrected calibration factor exceeds the lower limit of the correction threshold range, the corrected calibration factor is re-corrected to the lower limit of the correction threshold range.

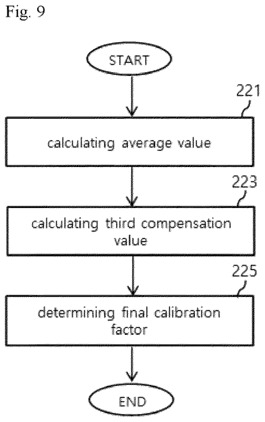

FIG. 9 is a flowchart to explain another example of the step of re-correcting the corrected calibration factor in the present disclosure.

Looking more specifically with reference to FIG. 9, the average value of the correction factor calculated in the third section is calculated based on the time when the reference biometric value is input (S221), and the third compensation value is calculated based on the calculated average value (S223).

Here, the third compensation value can be calculated by considering the difference between the corrected calibration factor and the average value so that the corrected calibration factor follows the average value, and the third compensation value can be calculated as in Equation (3) below.

[Equation 3]

$$CF_{R2} = CF_R - (CF_R - CF_M) \times \alpha_3$$

Here, CFR2, CFR, CFM, and $\alpha 3$ mean the final calibration factor, corrected calibration factor, average value, and the third weight, respectively, and $(CF_R - CF_m) \times \alpha_3$ refers to the third compensation value.

Here, the third weight can be set to 0.7 to 0.9.

For example, if the third weight is set to 0.8, the corrected calibration factor is 10, and the average value is 8, the final correction factor may be re-corrected to follow the average value.

Meanwhile, the above-described embodiments of the present disclosure can be written as a program that can be executed on a computer, and can be implemented in a general-purpose digital computer that operates the program using a computer-readable recording media.

The computer-readable recording media include storage media such as magnetic storage media (e.g., ROM, floppy disk, hard disk, etc.), optical reading media (e.g., CD-ROM, DVD, etc.), and carrier wave (e.g., transmission via Internet).

The present disclosure has been described with reference to the embodiments shown in the drawings, but these are merely examples, and those skilled in the art will understand that various modifications and other equivalent embodiments are possible therefrom. Therefore, the true scope of technical protection of the present disclosure should be determined by the technical concepts of the attached registration claims.

## Claims

1. A method for calibrating a biometric signal, comprising:

   determining whether a reference biometric value used to calculate a calibrated biometric value from a biometric signal measured by a sensor is input;
   extracting a calibration parameter at a time point when the reference biometric value is input, and determining whether a correction condition is satisfied based on the extracted calibration parameter; and
   calculating a calibration factor depending on whether the correction condition is satisfied and calculating the calibrated biometric value from the biometric signal measured by the sensor using the calibration factor.

2. The method for calibrating the biometric signal according to claim 1,
   wherein, if the correction condition is not satisfied, a current calibration factor is calculated from the input reference biometric value, and the calibrated biometric value is calculated from the biometric signal using the current calibration factor until a next reference biometric value is input.

3. The method for calibrating the biometric signal according to claim 1, comprising:

   if the correction condition is satisfied, calculating a current correction factor from the input reference biometric value;
   determining a calibration section to which the time point when the reference biometric value is input belongs;
   calculating a final calibration factor from the current calibration factor differently depending on the calibration section to which the time point when the reference biometric value is input belongs, based on the determined calibration section; and
   calculating a calibrated biometric value using the biometric signal measured by the sensor until the next reference biometric value is input and the final calibration factor.

4. The method for calibrating the biometric signal according to claim 3,
   wherein the calibration section divides an entire usage period of the sensor into a first section, a second section, and a third section over time based on time of inserting the sensor into a body.

5. The method for calibrating the biometric signal according to claim 4,
   wherein the current calibration factor is calculated as the final calibration factor when the time point when the reference biometric value is input is within the first section.

6. The method for calibrating the biometric signal according to claim 4,
   wherein, when the calibration section to which the time point when the reference biometric value is input is within the second section, a first compensation value is calculated from a difference between the current calibration factor and a previous calibration factor, and the final calibration factor is calculated by applying the first compensation value to the current calibration factor.

7. The method for calibrating the biometric signal according to claim 4,
   wherein, when the calibration section to which the time point when the reference biometric value is input is within the third section, a second compensation value is calculated from a difference between the current calibration factor and the previous calibration factor, and the final calibration factor is calculated by applying the second compensation value to the current calibration factor.

8. The method for calibrating the biometric signal according to claim 7, comprising:

   calculating an average value of previous final calibration factors calculated in the third section based on the time point when the reference biometric value is input; and
   re-correcting the final calibration factor based on the average value.

9. The method for calibrating the biometric signal according to claim 8,
   wherein the re-correcting of the final calibration factor comprises:

   calculating a correction threshold range based on the average value;
   determining whether the final calibration factor is out of the correction threshold range; and
   re-correcting the final calibration factor, depending on whether the final calibration factor is out of the correction threshold range, and
   wherein the calibrated biometric value is calculated using the re-corrected final calibration factor.

10. The method for calibrating the biometric signal according to claim 9,
   wherein, if the final calibration factor is out of the correction threshold range, the final calibration factor is re-corrected to the correction threshold range.

**11.** The method for calibrating the biometric signal according to claim 9, wherein, if the final calibration factor is within the correction threshold range, the final correction factor is used as is.

**12.** The method for calibrating the biometric signal according to claim 8,

wherein the re-correcting of the final calibration factor comprises calculating a difference between the final calibration factor and the average value; calculating a third compensation value from the difference between the final calibration factor and the average value; and re-correcting the final calibration factor with the third compensation value, and wherein the calibrated biometric value is calculated using the re-corrected final calibration factor.

**13.** The method for calibrating the biometric signal according to one of claims 1 to 12, wherein the sensor is configured to be inserted into the body of a user and continuously measure the biometric signal from body fluid for a certain period of time.

**14.** The method for calibrating the biometric signal according to one of claims 1 to 12,

wherein the sensor is configured to measure a blood glucose value of a user, and the reference biometric value is a reference blood glucose value measured by a separate blood glucose meter for calibrating the measured blood glucose value measured by the sensor.

Fig. 1

Fig. 2

**173**

CALIBRATION
CONDITION
DETERMINATION
UNIT

**175**

CALIBRATION
CONDITION
DETERMINATION
UNIT

**171**

CALIBRATION
FACTOR
DETERMINATION
UNIT

**178**

CALIBRATION
FACTOR
CORRECTION
UNIT

**177**

CALIBRATION
FACTOR
CALCULATION
UNIT

**179**

AVERAGE VALUE
CALCULATION
UNIT

Fig. 3

START

110
inputting reference blood
glucose value?

NO

YES    130

determining calibration section

150

calculating calibration factor

170

calculating calibrated blood
glucose value

END

Fig. 4

START

inputting reference blood glucose value? **110**

NO

YES **130**

determining calibration section

**150**

calculating calibration factor

**170**

calculating calibrated blood glucose value

END

Fig. 5

Fig. 6

Fig. 7

Fig. 8

START

calculating average value    211

calculating threshold range    213

within threshold range?    215

NO

re-correcting calibration factor    219

YES

determining final calibration
factor    217

END

Fig. 9

## INTERNATIONAL SEARCH REPORT

| International application No. |
|---|
| **PCT/KR2023/000397** |

**A. CLASSIFICATION OF SUBJECT MATTER**

**A61B 5/1495**(2006.01)i; **A61B 5/145**(2006.01)i; **A61B 5/155**(2006.01)i

According to International Patent Classification (IPC) or to both national classification and IPC

**B. FIELDS SEARCHED**

Minimum documentation searched (classification system followed by classification symbols)

A61B 5/1495(2006.01); A61B 5/00(2006.01); A61B 5/05(2006.01); A61B 5/145(2006.01); A61B 5/1455(2006.01); G01N 21/35(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 기준 (standard), 생체 (physiology), 파라미터 (parameter), 교정 (correct), 혈당 (blood glucose)

**C. DOCUMENTS CONSIDERED TO BE RELEVANT**

| Category* | Citation of document, with indication, where appropriate, of the relevant passages | Relevant to claim No. |
|---|---|---|
| Y | EP 2840958 B1 (ABBOTT DIABETES CARE, INC.) 30 September 2020 (2020-09-30)<br>See claim 1. | 1,2,13,14 |
| A | | 3-12 |
| Y | KR 10-2035424 B1 (UNIST(ULSAN NATIONAL INSTITUTE OF SCIENCE AND TECHNOLOGY)) 22 October 2019 (2019-10-22)<br>See paragraphs [0055], [0057] and [0063]. | 1,2,13,14 |
| Y | KR 10-2021-0064651 A (I-SENS, INC.) 03 June 2021 (2021-06-03)<br>See paragraphs [0012] and [0043]. | 13,14 |
| A | US 2010-0168537 A1 (UEDA et al.) 01 July 2010 (2010-07-01)<br>See entire document. | 1-14 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **20 April 2023** | **20 April 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2023/000397** |

**C.    DOCUMENTS CONSIDERED TO BE RELEVANT**

| Category* | Citation of document, with indication, where appropriate, of the relevant passages | Relevant to claim No. |
| --- | --- | --- |
| A | JP 2011-062335 A (PANASONIC ELECTRIC WORKS CO., LTD.) 31 March 2011 (2011-03-31)<br>See entire document. | 1-14 |

Form PCT/ISA/210 (second sheet) (July 2022)

**INTERNATIONAL SEARCH REPORT**
Information on patent family members

International application No.

**PCT/KR2023/000397**

| Patent document cited in search report | | | Publication date (day/month/year) | Patent family member(s) | | | Publication date (day/month/year) |
|---|---|---|---|---|---|---|---|
| EP | 2840958 | B1 | 30 September 2020 | EP | 2840958 | A1 | 04 March 2015 |
| | | | | EP | 3777664 | A1 | 17 February 2021 |
| | | | | US | 10561372 | B2 | 18 February 2020 |
| | | | | US | 2013-0304389 | A1 | 14 November 2013 |
| | | | | US | 2017-0095213 | A1 | 06 April 2017 |
| | | | | US | 2020-0146634 | A1 | 14 May 2020 |
| | | | | US | 9462970 | B2 | 11 October 2016 |
| | | | | WO | 2013-163342 | A1 | 31 October 2013 |
| KR | 10-2035424 | B1 | 22 October 2019 | | None | | |
| KR | 10-2021-0064651 | A | 03 June 2021 | AU | 2020-393447 | A1 | 14 October 2021 |
| | | | | AU | 2020-393447 | B2 | 13 October 2022 |
| | | | | EP | 3932310 | A1 | 05 January 2022 |
| | | | | JP | 2022-530511 | A | 29 June 2022 |
| | | | | JP | 7247370 | B2 | 28 March 2023 |
| | | | | KR | 10-2022-0130646 | A | 27 September 2022 |
| | | | | KR | 10-2445697 | B1 | 23 September 2022 |
| | | | | US | 2022-0160266 | A1 | 26 May 2022 |
| | | | | WO | 2021-107335 | A1 | 03 June 2021 |
| US | 2010-0168537 | A1 | 01 July 2010 | US | 8323195 | B2 | 04 December 2012 |
| JP | 2011-062335 | A | 31 March 2011 | | None | | |

Form PCT/ISA/210 (patent family annex) (July 2022)